# FASCICULE DE BREVET EUROPEEN

(11) **EP 0 700 992 B1**
(45) Date de publication et mention de la délivrance du brevet: **15.01.2003**
(21) Numéro de dépôt: 95402004.6
(22) Date de dépôt: 05.09.1995
(51) Int. Cl.: C12N 1/10, C12N 1/36, C12Q 1/68, A61K 39/018

(54) **Procédés de culture, d'atténuation de la virulence et de clonage in vitro de parasites du genre Babesia et leurs applications**
Verfahren zur Kultivierung, Virulenzabschwächung und in vitro Klonierung des Parasiten aus der Gattung Babesien und ihre Anwendungen
Method of cultivation, attenuation of virulence and the in vitro cloning of the genus Babesia and their applications

(30) Priorité: 08.09.1994 FR 9410762
(43) Date de publication de la demande: 13.03.1996
(73) Titulaire: VIRBAC, 06516 Carros (FR)
(72) Inventeur: Mahl, Philippe, F-06270 Villeneuve-Loubet (FR); Dick-Madelpuech, Marie-Hélène, F-75015 Paris (FR)
(74) Mandataire: Orès, Bernard

(56) Documents cités:
- EP-A- 0 018 579
- EP-A- 0 220 988
- WO-A-84/01716
- WO-A-93/14204
- FR-A- 2 655 853
- GB-A- 2 062 463
- INTERNATIONAL PARASITOLOGY, vol. 23, no. 6, PERGAMON PRESS,GB, pages 771-776, B. HENTRICH AND R. BÖSE 'Cryopreservation of Babesia divergens from jirds as a live vaccine for cattle'
- VETERINARY PARASITOLOGY, vol. 31, no. 3,4, ELSEVIER, AMSTERDAM, NL, pages 243-251, W.K. JORGENSEN ET AL. 'Infectivity of cryopreserved Babesia bovis, Babesia bigemina and Anaplasma centrale for cattle after thawing, dilution and incubation at 30 C'
- TRENHOLME AND PHILLIPS: 'The use of feeder cells in the cultivation of Plasmodium falciparum asexual blood stages' PARASITOLOGY RESEARCH vol. 75, 1989, pages 518 - 521

### LISTE DE SEQUENCES

(1) INFORMATIONS GENERALES:
   (i) DEPOSANT:
      (A) NOM: LABORATOIRES VIRBAC
      (B) RUE: 1ERE AVENUE 2065 m - LID
      (C) VILLE: CARROS
      (E) PAYS: FRANCE
      (F) CODE POSTAL: 06516
   (ii) TITRE DE L' INVENTION: PROCEDES DE CULTURE, D'ATTENUATION DE LA VIRULENCE ET DE CLONAGE IN VITRO DE PARASITES DU GENRE BABESIA ET LEURS APPLICATIONS.
   (iii) NOMBRE DE SEQUENCES: 1
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 606 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 1:
   AAGTGATACC TGCCACTGCA TCCTTACACG ATAGACTAGC ATTATGATCC CAGCACATGT    60
   TGCAGCACGT ATACGAAACA TATACGTCGT AATGTGACGC CAGATTCACA TGTAGCATGG   120
   GCATCATCCC GATCGCCACG CTGGCGTCAG GCCTGGTGCC TCATCACAAT CACGTTCGGA   180
   TTATCATGAT ATATTCAATA CATTCATGAT ATAGGCATCA TTCTGACCAC AATATGCGTC   240
   CTGTAACGGA TAGGATGCTG TATCTGATGT GGTTCCACTA CATGTTACCT GCCCCGCTTA   300
   GGCCCATACC CAGGTGACCT GAGGTCACGA CCACGTGAGC CAAATTTGGC CATCCAGGAT   360
   GCAAGTTAGA TTATCGCAGA AAAGAGTCAT CAAGCTCATA TAAAGCGTGT TGGCGCCATT   420
   CTAAGCGTAG TTACGCTTGG CTGCGCTGGG CTGTACCACA GGTAACAAAT CCTGAATCAC   480
   AGTATCAAGC TGTTGCTTAA AGTCACTTCC TCCATTATTC TGATTAGCCA CACCATCAAT   540
   ATCCTTTAAT TCGGTCTTAA GTCCATCCAC CACCTTGGTC TTCCCCTTAC CACTGGTGTT   600
   CCACAA                                                              606

## Description

La présente Invention se rapporte à un procédé de culture *in vitro* de parasites du genre *Babesia,* à un procédé d'atténuation *in vitro* de la virulence de ces parasites, à un procédé de clonage *in vitro* de ces mêmes parasites, à des parasites de virulence atténuée et à des lignées clonales du genre *Babesia,* à des sondes nucléotidiques issues de l'ADN de ces lignées clonales, à l'utilisation de ces sondes pour l'identification de parasites *Babesia*, à des vaccins vivants atténués contre les babésioses ainsi qu'aux applications des procédés ci-dessus mentionnés.

Les babésioses, encore appelées piroplasmoses, sont des parasitoses intraérythrocytaires, transmises par les morsures de tiques, qui affectent de nombreuses espèces animales domestiques et sauvages et dont l'agent est un protozoaire du genre *Babesia.*

Il existe ainsi :
* une babésiose bovine causée par *Babesia bovis*, *Babesia bigemina, Babesia major* et *Babesia divergens* qui est responsable de pertes économiques importantes et représente un obstacle majeur à l'augmentation de la productivité de l'élevage bovin, notamment dans les pays en voie de développement ;
* une babésiose canine due à *Babesia canis* et *Babesia gibsoni* et qui, chaque année, tue de nombreux chiens ou laisse des séquelles graves chez un certain nombre d'entre eux ;
* une babésiose équine due à *Babesia equi* et *Babesia caballi* ;
* une babésiose ovine due à *Babesia ovis* ;
* une babésiose des rongeurs causée par *Babesia microti* ;
* une babésiose humaine, rare mais sévère puisqu'elle se traduit par un décès chez plus de la moitié des sujets. Les espèces de *Babesia* en cause chez l'homme sont habituellement *Babesia divergens* et *Babesia microti.*

La préparation de vaccins contre les babésioses a, pour ces raisons, fait l'objet de nombreux travaux de recherche au cours de ces 20 dernières années.

Plusieurs équipes se sont orientées vers l'extraction et la purification des principes antigéniques en vue de leur utilisation pour la fabrication de vaccins. Ainsi, ont été proposés :
* dans EP-A-18 579, un procédé de propagation *in vitro* des *Babesia* visant à obtenir, dans le milieu de culture, un antigène soluble, spécifique de ces parasites et utilisable à des fins immunitaires ou diagnostiques. Ce procédé utilise une multiplication des *Babesia* dans des érythrocytes placés dans un milieu comprenant 30 à 50% de sérum défibriné et maintenus en atmosphère enrichie en CO₂ (3 à 6%) et contrôlée en O₂ pour maintenir l'hémoglobine des érythrocytes à l'état désoxydé.

Ce procédé s'est révélé peu adapté à la multiplication de certaines espèces de *Babesia,* comme par exemple, *Babesia canis.*
* dans GB-A-2 062 463, une méthode consistant à désintégrer par ultra-sons des érythrocytes infestés par *Babesia* et à extraire de la fraction soluble de la suspension ainsi obtenue l'antigène spécifique.
* dans EP-A-220 988, un procédé d'incubation *in vitro* des parasites *Babesia canis* utilisant des érythrocytes infestés dans un milieu approprié et permettant de recueillir dans le surnageant les antigènes spécifiques. Cette culture est réalisée en atmosphère normale, c'est à dire composée essentiellement d'oxygène et d'azote et dépourvue d'une teneur importante en gaz carbonique. Elle utilise deux incubations, la première d'environ 8 heures à une température de 34-38°C, la seconde d'environ 16 heures à une température comprise entre 0 et 10°C.

Bien que ce procédé constitue un progrès certain sur les techniques antérieures, il présente l'inconvénient de nécessiter, pour initialiser la culture, un sang très enrichi en parasites et, par voie de conséquence, d'infester expérimentalement des chiens splénectomisés. En outre, le taux de multiplication des parasites dans ces conditions de culture reste faible, dépassant à peine le coefficient de survie des parasites. Cette difficulté est d'ailleurs rapportée dans la Demande Internationale WO 91/08771 au nom de la même titulaire qui souligne que, dans les cultures de *Babesia divergens* et *Babesia canis,* la parasitémie chute à moins de 1% après 3 semaines, de sorte qu'il convient d'initialiser de nouvelles cultures et, de ce fait, d'infester à nouveau des animaux splénectomisés.

Le document WO 84/01716 décrit un procédé de préparation de parasites de virulence atténuée de l'espèce *Babesia bovis,* ce procédé consistant à cultiver le parasite dans un milieu comprenant des érythrocytes infectés et des érythrocytes non infectés par *Babesia bovis.*

Ce procédé comporte des dilutions successives et la sélection de lignées clonales avirulentes et à la croissance rapide.

Le procédé décrit dans ce document nécessite d'avoir recours à la splénectomie.

Le document Treholme and Phillips (1989), Parasitology Research 75,518-521, décrit un procédé de culture *in vitro* de parasites du genre *Plasmodium falciparum* comprenant l'incubation d'hématies parasitées en présence de cellules de support.

Toutefois, rien n'incite l'homme du métier à combiner l'enseignement de ce document avec celui des autres documents de l'art antérieur.
* dans FR-A-2 608 926, une méthode d'extraction d'un antigène d'environ 48.000 daltons, spécifique de *Babesia* *divergens*, à partir de sang de boeuf fortement infesté, en vue de son utilisation pour la préparation de vaccins.

Plus récemment, un certain nombre de chercheurs se sont orientés sur la production de protéines recombinantes par identification et clonage des séquences d'ADN codant pour les antigènes spécifiques des *Babesia* et transformation de cellules hôtes appropriées telles qu'*Escherichia coli*. Ces méthodes sont décrites par exemple dans les Demandes EP-A-382 012 et EP-A-417 524 ou encore dans la Demande Internationale WO 91/11776.

Séduisantes sur le plan théorique mais relativement coûteuses, elles apparaissent difficilement exploitables industriellement pour la production de vaccins à grande échelle et aucune d'entre elles n'a encore abouti.

En outre, l'immunisation à partir de principes antigéniques s'avère souvent être incomplète, notamment vis-à-vis de souches hétérologues et parfois même vis-à-vis de souches homologues, comme le montrent par exemple les tests de vaccination décrits dans EP-A-382 012.

Le document WO84/01716 décrit un procédé de préparation d'un vaccin vivant contre la babésiose bovine, ce vaccin étant préparé à partir d'érythrocytes de bovin infectés par une population clonée de *Babesia bovis.* La souche clonale est préparée par la culture en dilutions successives d'un mélange de *Babesia* virulent et avirulent et la sélection d'un clone avirulent à croissance rapide. Toutefois, ce procédé nécessite de recourir à la splénectomie.

La Demanderesse s'est, en conséquence, fixé pour but de fournir un procédé permettant d'atténuer *in vitro* la virulence de parasites du genre *Babesia* de sorte à pouvoir utiliser ces parasites pour la fabrication de vaccins vivants atténués et qui soit applicable aux différentes espèces de *Babesia.*

La Demanderesse s'est, également, fixé pour but de fournir un procédé de culture *in vitro* des parasites du genre *Babesia* qui permette de façon simple et peu coûteuse de produire ces parasites à grande échelle pour la préparation de vaccins contre les babésioses et qui ne nécessite à aucun moment de recourir à une splénectomie chez des animaux.

La Demanderesse s'est, aussi, fixé pour but de pourvoir à un vaccin vivant atténué, propre à conférer une protection spécifique contre une espèce de *Babesia* et ce, quelle que soit l'infestation par ladite espèce (souches homologues ou hétérologues), sans transformer les sujets vaccinés en réservoirs d'agents pathogènes et dont les contraintes de production soient compatibles avec une exploitation industrielle.
La présente Invention a pour objet un procédé de culture *in vitro* de parasites du genre *Babesia* comprenant au moins une incubation d'hématies parasitées par lesdits parasites en présence d'hématies homologues non parasitées dans un milieu de culture convenable et des conditions de culture appropriées, caractérisé en ce que la ou lesdites incubations sont réalisées en présence de cellules propres à servir de support à la culture desdits parasites.

La Demanderesse a, en effet, découvert que la culture *in vitro* de parasites *Babesia* par incubation d'hématies parasitées en présence d'hématies non parasitées dans un milieu de culture approprié permet d'obtenir une croissance très productive de ces parasites lorsque cette culture est réalisée en présence de cellules aptes à servir de support à ladite culture des parasites, par exemple, par la formation d'une sous-couche.

Dans un mode de mise en oeuvre préféré du procédé conforme à l'Invention, lesdites cellules sont des cellules animales issues de lignées immortelles telles que, par exemple, les cellules C32 (cellules de mélanome humain), les cellules MDCK (cellules rénales de chien), les cellules BHK21 (cellules rénales de nouveaux-nés de hamster) ou les cellules AK-D (cellules pulmonaires de foetus de chat).

A titre d'exemples, les cellules C32, MDCK et AK-D sont disponibles auprès de l'AMERICAN TYPE CULTURE COLLECTION dans laquelle elles sont respectivement identifiées sous les références CRL 1585, CCL 34 et CCL 150.

Toutefois, la Demanderesse a pu vérifier que d'autres cellules animales telles que, par exemple, des monocytes ou des lymphocytes permettent de mettre en oeuvre le procédé de culture conforme à l'Invention.

L'incubation des hématies est réalisée en présence d'une quantité suffisante desdites cellules pour permettre la croissance des parasites, ces cellules étant, de préférence, non confluentes.

Selon une disposition avantageuse du procédé conforme à l'Invention, le milieu de culture est un milieu liquide du type RPMI 1640, MEM, M199, WILLIAMS ou autre, additionné de 2 à 40% de sérum homologue ou hétérologue décomplémenté et/ou d'un substitut de sérum et, facultativement, de facteurs de croissance tels que l'hypoxanthine, d'antibiotiques tels que la gentamycine, la pénicilline ou la streptomycine, d'antifongiques ou d'antioxydants, ledit milieu de culture étant en outre tamponné de façon à maintenir le pH entre 7 et 7,7.

La culture des parasites peut, toutefois, être également réalisée dans un milieu semi-liquide.

Conformément à la présente Invention, le rapport entre le volume des hématies présentes dans la culture et le volume du milieu de culture est compris entre 0,1 et 50%, avantageusement entre 2,5 et 10% et, de préférence, égal à 5%.

L'incubation des hématies est réalisée à une température comprise entre 30 entre 40°C et, préférentiellement, proche de 37°C.

Conformément à l'Invention, le procédé de culture comprend, pour la culture en continue des parasites, plusieurs incubations successives.

Il est, en effet, possible de réaliser une culture en continue des parasites selon le principe des subcultures, c'est à dire par l'incubation, dans de nouveaux puits de culture, d'une dilution des hématies parasitées recueillies au terme d'une incubation antérieure, ladite dilution correspondant à l'adjonction des hématies parasitées dans des hématies non parasitées pour abaisser la parasitémie.

Selon une disposition particulièrement avantageuse du procédé conforme à l'Invention, la première incubation peut être réalisée avec des hématies parasitées ou des zoïtes obtenus à partir de sang d'animaux infectés à *Babesia.*

Conformément à la présente Invention, le procédé de culture comprend en outre la conservation à basse température des hématies parasitées dans un milieu comprenant un cryopréservant tel que, par exemple, le diméthylsulfoxyde et une macromolécule à fort pouvoir osmotique telle que, par exemple, la polyvinylpyrrolidone.

Il est possible de cultiver par le procédé de culture conforme à l'Invention les différentes espèces de *Babesia,* et notamment *Babesia canis, Babesia gibsoni, Babesia bovis, Babesia bigemina, Babesia major, Babesia divergens, Babesia equi, Babesia caballi, Babesia ovis* et *Babesia microti.*

Le procédé de culture conforme à l'Invention trouve application à la production en grand nombre de parasites du genre *Babesia.*

Il trouve également application à la détection d'une infection asymptomatique à *Babesia.*

La présente Invention a, également, pour objet un procédé d'atténuation *in vitro* de la virulence de parasites du genre *Babesia,* caractérisé en ce qu'il comprend la culture desdits parasites selon le procédé de culture *in vitro* conforme à l'Invention pendant au moins 8 jours.

La Demanderesse a, en effet, découvert qu'en cultivant des parasites *Babesia* selon le procédé de culture conforme à l'Invention pendant un temps suffisant, on obtient une atténuation, voire une disparition de la virulence desdits parasites.

Dans un mode de mise en oeuvre préféré du procédé d'atténuation conforme à l'Invention, la culture des parasites est réalisée pendant un temps compris entre 20 et 60 jours.

L'atténuation de la virulence des parasites peut être contrôlée par l'injection des parasites de la culture en cours à un animal sensible.

Ce procédé permet d'atténuer la virulence de parasites de différentes espèces de *Babesia,* et notamment de *Babesia canis, Babesia gibsoni*, *Babesia bovis, Babesia* *bigemina, Babesia major, Babesia divergens, Babesia equi, Babesia caballi, Babesia ovis* et de *Babesia microti.*

Le procédé d'atténuation conforme à l'Invention trouve notamment application à la préparation de vaccins vivants atténués.

La présente Invention a, également, pour objet des parasites du genre *Babesia* de virulence atténuée autres qu'appartenant aux espèces *Babesia bovis, Babesia bigemina* et *Babesia divergens.*

Dans une forme de réalisation préférée de l'Invention, les parasites de virulence atténuée appartiennent à l'espèce *Babesia canis.*

La présente Invention a, aussi, pour objet un procédé de clonage *in vitro* de parasites du genre *Babesia*, comprenant :
a) l'isolement d'un seul parasite à partir d'une suspension d'hématies parasitées par lesdits parasites ou d'une population de zoïtes,
b) la culture du parasite isolé par le procédé de culture *in vitro* conforme à l'Invention,
les étapes a) et b) étant effectuées une ou plusieurs fois jusqu'à l'obtention d'une lignée homogène de parasites.

Conformément à l'Invention, l'isolement du parasite peut être réalisée par une dilution limite de la suspension d'hématies parasitées ou de la population de zoïtes ou au moyen d'un trieur de cellules ou encore par micromanipulation.

Par dilution limite, on entend au sens de la présente Invention une dilution suffisamment haute de la suspension d'hématies parasitées ou de la population de zoïtes utilisées de sorte que, selon toute probabilité ne soit introduit dans chaque puits de culture que 0 ou 1 hématie parasitée ou zoïte et, qu'en conséquence, se développe dans chaque puits une lignée homogène issue d'un seul parasite.

Par micromanipulation, on entend l'aspiration dans une micropipette d'une seule hématie parasitée sous contrôle visuel.

Il est possible d'obtenir, par le procédé conforme à l'Invention, des lignées clonales de nombreuses espèces *Babesia,* et notamment de *Babesia canis, Babesia gibsoni, Babesia bovis, Babesia bigemina, Babesia major, Babesia divergens, Babesia equi, Babesia caballi, Babesia ovis* et de *Babesia microti.*

Le procédé de clonage conforme à l'Invention trouve notamment application à la préparation de vaccins vivants atténués.

La présente Invention a, également, pour objet une lignée clonale de parasites du genre *Babesia* autres qu'appartenant à l'espèce *Babesia bovis.*

Dans une forme de réalisation préférée de l'Invention, la lignée clonale est une lignée de parasites appartenant à l'espèce *Babesia canis.*

Selon une disposition avantageuse de ce mode de réalisation, la lignée clonale est la lignée désignée ci-après PIII de *Babesia canis*, déposée auprès de l'EUROPEAN COLLECTION OF ANIMAL CELL CULTURES en date du 6 Septembre 1994 et identifiée dans cette Collection sous le n° 94090611.

La présente Invention a, aussi, pour objet une sonde oligonucléotidique, caractérisée en ce qu'elle est issue de l'ADN de parasites de la lignée clonale PIII.

De façon avantageuse, la sonde oligonucléotidique présente tout ou partie de la séquence suivante :

La présente Invention a, par ailleurs, pour objet l'utilisation de cette sonde oligonucléotidique pour identifier des parasites du genre *Babesia.*

Selon une disposition avantageuse, cette sonde oligonucléotidique permet, en outre, de différencier les souches et/ou lignées clonales de parasites du genre *Babesia.*

La présente Invention a, également, pour objet un vaccin vivant atténué contre une babésiose, caractérisé en ce qu'il comprend des hématies parasitées par des parasites du genre *Babesia* autres qu'appartenant à aux espèces *Babesia bovis, Babesia bigemina* et *Babesia divergens* dans un véhicule pharmacologiquement acceptable.

Dans une forme de réalisation préférée de l'Invention, le vaccin est propre à induire une protection efficace contre la babésiose canine à *Babesia canis* et est constitué d'hématies parasitées par la lignée clonale PIII de *Babesia canis.*

L'Invention a, également, pour objet un vaccin vivant atténué contre une babésiose, caractérisé en ce qu'il est constitué d'hématies parasitées par des parasites du genre *Babesia* obtenus par l'un quelconque des procédés précédemment décrits.

Un tel vaccin pourra, en effet, être préparé :
- soit avec des hématies parasitées issues de la culture d'une souche de parasites naturellement avirulents ou peu virulents mise en oeuvre selon le procédé de culture conforme à l'Invention ;
- soit avec des hématies parasitées issues de la culture d'une souche ayant naturellement une virulence plus ou moins prononcée mais dont la virulence aura été préalablement atténuée par le procédé d'atténuation conforme à l'Invention ;
- soit, enfin, de la culture d'une lignée clonale obtenue par le procédé de clonage conforme à l'Invention et ayant une virulence extrêmement faible.

L'Invention sera mieux comprise à l'aide de la description qui suit, faite à titre d'exemple et en référence aux dessins annexés dans lesquels :
- les Figures 1A et 1B montrent la croissance de parasites *Babesia canis* obtenue respectivement par le procédé de culture *in vitro* conforme à l'Invention et par un procédé de culture de l'Art Antérieur ;
- les Figures 2A et 2B montrent l'évolution de l'hématocrite et de la parasitémie chez des chiens inoculés respectivement avec une souche virulente de *Babesia canis* et la même souche après atténuation par le procédé d'atténuation conforme à l'Invention ;
- les Figures 3A et 3B montrent l'évolution de l'hématocrite et de la parasitémie chez des chiens, préalablement vaccinés respectivement par un vaccin préparé à partir de la lignée clonale PIII et par un vaccin de l'Art Antérieur, après administration d'une souche virulente de *Babesia canis ;*
- la Figure 4 représente le profil des fragments de restriction, obtenu après une double digestion par les enzymes de restriction *Ase*I/*Bam*HI, de l'ADN génomique de la lignée clonale PIII de *Babesia canis ;*
- la Figure 5 représente les profils des fragments de restriction, obtenus après une double digestion par les enzymes de restriction *Ase*I/*Bam*HI*,* de souches de *Babesia canis* issues de chiens souffrant d'une babésiose ;
- la Figure 6 représente la croissance de parasites *Babesia canis* obtenue par une variante du procédé de culture *in vitro* conforme à l'Invention.

### EXEMPLE 1 : CULTURE DES CELLULES DESTINEES A SERVIR DE SUPPORT A LA CULTURE DES PARASITES

Le présent Exemple utilise des cellules AK-D capables de se multiplier dans un milieu de culture approprié et propre à être utilisées comme sous-couches dans la culture de parasites *Babesia.*

### 1) Origine des cellules AK-D

Les cellules AK-D proviennent de la souche déposée auprès de l'AMERICAN TYPE CULTURE COLLECTION sous le numéro CCL 150.

Ces cellules sont conservées par congélation selon le protocole suivant :
après un comptage sur cellule de numération, les cellules sont centrifugées à 1200g pendant 5 minutes. Le surnageant est éliminé. Du milieu de congélation (RPMI 1640/SVF/DMSO - v/v: 75/15/10) est ajouté au culot cellulaire dans un volume approprié pour obtenir une concentration cellulaire égale à 2.10⁶ cellules/ml. La suspension cellulaire ainsi obtenue est répartie en échantillons d'1 ml qui sont conservés dans l'azote liquide.

### 2) Milieu de culture des cellules AK-D

Les cellules AK-D sont cultivées dans un milieu de culture liquide ayant la composition ci-après :
- sérum de veau foetal 5 ml
- glutamine 2mM
- gentamycine 2,5 mg
- milieu RPMI 1640 complémenté q.s.p. 50 ml
Ce milieu est filtré sur une membrane de 0,22 µm.

Au sens de la présente Invention, on entend par milieu RPMI 1640 complémenté un milieu liquide présentant la composition ci-après:
- poudre de RPMI 1640 10,4 g
- HEPES 8,3 g
- NaHCO₃ 2 g
- hypoxanthine 0,001 g
- Eau q.s.p. 1 litre
Ce milieu est filtré sur une membrane de 0,22 µm.

### 3) Préparation de sous-couches cellulaires à partir de cellules AK-D congelées

Un échantillon d'1 ml contenant 2.10⁶ cellules AK-D congelées est soumis à une décongélation rapide à 37°C. Les cellules sont mises en suspension dans un tube contenant 10 ml du milieu de culture décrit ci-avant. Le tube est soumis à une centrifugation à 400g pendant 10 minutes. Le surnageant est éliminé et le culot est repris dans 5 ml de milieu de culture dans un flacon de culture cellulaire de 25 cm². On laisse les cellules pousser jusqu'à confluence, soit environ 3 jours.

Lorsque les cellules sont confluentes, le surnageant est écarté et les cellules sont lavées rapidement deux fois avec 5 ml de milieu RPMI complémenté. 2 ml d'une solution de trypsine-EDTA 0,25% sont ajoutés. La séparation des cellules est suivie au microscope. Lorsque les cellules sont dissociées les unes des autres, la suspension cellulaire est reprise dans du milieu de culture, centrifugée à 400g pendant 10 minutes et le culot obtenu est repris dans 5 ml de milieu de culture. Il est alors possible de procéder à un comptage des cellules AK-D sur cellule de numération à l'aide d'une goutte de suspension cellulaire.

Cette suspension cellulaire peut être entretenue par des repiquages successifs dans des flacons de culture cellulaire de 25 cm² à raison de 5 ml de milieu de culture par flacon et de 2.10⁵ cellules/ml de milieu de culture. Lors de chaque repiquage, on attend que les cellules poussent à confluence, soit environ 3 jours. Elles sont alors soumises à un traitement par une solution de trypsine-EDTA 0,25% comme décrit ci-avant à l'issue duquel elles peuvent être comptées et diluées de sorte à obtenir une nouvelle suspension comptant 2.10⁵ cellules/ml, utilisable pour un nouveau repiquage.

L'utilisation de cellules AK-D fraîchement décongelées pour servir de sous-couches dans une culture de parasites *Babesia* nécessite que la culture de cellules AK-D soit préalablement soumise à trois repiquages comme décrit ci-avant. A l'issue du troisième repiquage, les cellules AK-D peuvent être ensemencées dans les puits d'une plaque de culture cellulaire.

Pour ce faire, on dépose, dans chaque puits d'une plaque de 24 puits, 1 ml de suspension cellulaire renfermant 3.10⁴ cellules AK-D. L'ensemencement des *Babesia* peut être effectué sur ces cellules dès le lendemain.

### EXEMPLE 2 : CULTURE DE PARASITES BABESIA CANIS

La culture *in vitro* des parasites *Babesia canis* selon le procédé conforme à l'Invention peut être initiée au moyen d'hématies parasitées ou de zoïtes de *Babesia canis* obtenus à partir du sang de chiens infectés, fraîchement recueillis ou ayant été conservés à température ambiante ou par congélation.

Le présent Exemple utilise des hématies parasitées provenant de prélèvements sanguins effectués chez des chiens présentant une piroplasmose symptomatique à *Babesia canis* et ayant été conservées par congélation.

### 1) Traitement des prélèvements sanguins et conservation des hématies parasitées

Après centrifugation des échantillons sanguins à 1200g pendant 10 minutes, le volume des culots globulaires obtenus est mesuré. Les surnageants sont éliminés et les culots sont mélangés à un milieu de congélation (v/v : 1/1) présentant la composition suivante :
- DMSO (diméthylsulfoxyde) 40 g
- Polyvinylpyrrolidone 40 (SIGMA) 20 g
- RPMI 1640 complémenté q.s.p. 100 ml
et ayant été préalablement filtré sur une membrane de 0,45 µm.

Le mélange hématies - milieu de congélation est conservé dans l'azote liquide.

Le DMSO peut être avantageusement remplacé par un autre cryopréservant tel que, par exemple, le glycérol. La polyvinylpyrrolidone, macromolécule à haut pouvoir osmotique, peut être remplacée par d'autres composés de haut poids moléculaire également à haut pouvoir osmotique.

### 2) Préparation des hématies fraîches non parasitées

Les hématies non parasitées utilisées pour la culture des parasites *Babesia canis* sont isolées d'échantillons sanguins prélevés sur des chiens de race Beagle du groupe A⁻, élevés en conditions S.P.F (specific pathogen free) et exempts de toute infection à *Babesia.*

Les échantillons sanguins sont soumis à une centrifugation à 2200g pendant 10 minutes. Le plasma et les globules blancs sont éliminés. Les hématies sont lavées 2 fois avec 10 volumes de RPMI 1640 complémenté. A l'issue du deuxième lavage, le culot globulaire est repris dans un volume de RPMI 1640 complémenté propre à obtenir une suspension d'hématies fraîches non parasitées présentant un hématocrite de 50%.

### 3) Initialisation de la culture des Babesia canis à partir d'hématies parasitées congelées

Une ampoule contenant 200 µl d'un mélange congelé d'hématies parasitées et de RPMI 1640 complémenté est mise à décongeler dans un bain-marie de 37°C pendant 1 minute.

Dans l'ampoule, est ajouté 1 ml d'une solution de NaCl à 3,5% et l'ensemble est soumis à une centrifugation à 400g pendant 5 minutes.

Le surnageant est éliminé et le culot est repris dans 1 ml d'un milieu de culture liquide présentant la composition ci-après :
- sérum de chien 0,05 ml
- Nutridoma® SR 0,02 ml
- glutamine 2 mM
- gentamycine 50 µg
- RPMI 1640 complémenté q.s.p. 1 ml
pH = 7,3

Le sérum de chien entrant dans la composition du milieu de culture des parasites est disponible auprès de la Société EUROBIO. Avant d'être ajouté aux autres constituants de ce milieu de culture, il est décomplémenté à 56°C pendant 30 minutes, puis filtré à travers une membrane de 0,5 µm.

Le Nutridoma® SR est un substitut de sérum disponible auprès de la Société BOEHRINGER MANNHEIM.

On obtient ainsi une suspension d'hématies parasitées propre à être utilisée pour initialiser une culture *in vitro* de *Babesia canis.*

L'initialisation de cette culture est réalisée selon le mode opératoire suivant :
* à J0, on ensemence les parasites *Babesia canis* dans les puits d'une plaque de culture de 24 puits préalablement ensemencés de cellules AK-D en éliminant le milieu de culture des cellules AK-D présent dans les puits et en déposant dans chaque puits 1 ml de la suspension d'hématies parasitées préparée ci-avant ainsi que 50 µl d'une suspension d'hématies fraîches non parasitées préparée comme décrit au point 2) ci-avant.
   La plaque de 24 puits est alors placée dans une chambre d'incubation à bougie ou dans un incubateur à régulation d'O₂ et de CO₂, sous une atmosphère enrichie en CO₂ (3 à 7%) et appauvrie en O₂ (5 à 17%). La chambre à bougie est elle-même placée dans une étuve à 37°C.
   En milieu de journée, le milieu de culture, c'est à dire le liquide qui surnage les hématies sédimentées, est éliminé et remplacé par un volume identique de milieu de culture frais.
* le lendemain (J1) et le surlendemain (J2), le milieu de culture est remplacé par un volume identique de milieu de culture frais. 25 µl de suspension d'hématies fraîches non parasitées peuvent être ajoutés dans chaque puits.
* à J3, un frottis est réalisé :
   - si le frottis ne permet pas de révéler la présence de parasites, les cultures sont soumises à une dilution au 1/2 selon le mode opératoire décrit ci-après :
      * élimination du milieu de culture de la culture devant être soumise à dilution,
      * reprise des hématies de cette culture à l'aide de 200 µl de milieu de culture frais,
      * rejet de la moitié du volume ainsi obtenu dans un puits de culture,
      * ajout de 0,9 ml de milieu de culture liquide frais et de 50 µl de suspension d'hématies fraîches non parasitées.
         Cette dilution a pour effet d'apporter à la culture à la fois des hématies fraîches non parasitées et du milieu de culture frais.
   - si ce frottis révèle un pourcentage d'hématies parasitées supérieur ou égal à 0,2%, les cultures de parasites sont soumises à une dilution propre à ensemencer les puits d'une nouvelle plaque de culture avec une suspension d'hématies parasitées à 0,01% et à réaliser une culture en continue des parasites dans les conditions indiquées au point 4) ci-après.
* à J5, un nouveau frottis est réalisé sur les cultures dont le frottis était négatif à J3 :
   - si le frottis ne met pas en évidence la présence de parasites, le milieu de culture est changé sans dilution.
   - si le frottis permet de relever un pourcentage d'hématies parasitées supérieur ou égal à 0,2%, les cultures sont soumises à une dilution propre à permettre l'ensemencement des puits d'une nouvelle plaque de culture avec une suspension d'hématies parasitées à 0,01% et à réaliser une culture en continue des parasites.
* à J6, on procède à un nouveau frottis sur les cultures dont le frottis était négatif à J5. Selon le résultat de ce frottis, on procède à des dilutions comme à J3.
On continue ainsi jusqu'à J10 et, dans le cas où aucune parasitémie n'est alors décelable, il convient d'initier une nouvelle culture à partir d'une autre ampoule d'hématies parasitées.

Dans tous les cas, chaque culture de *Babesia canis* doit être transférée tous les 2 jours sur une nouvelle sous-couche de cellules AK-D.

### 4) Conditions de culture en continue des parasites Babesia canis

La culture en continue *in vitro* des parasites *Babesia canis* est réalisée selon le principe des subcultures, chaque subculture consistant à incuber dans de nouveaux puits de culture une dilution des hématies parasitées recueillies au terme d'une incubation antérieure.

Pour des subcultures réalisées dans des plaques de 24 puits, les conditions ci-dessous se sont révélées particulièrement avantageuses :
- dépôt des hématies parasitées dans des puits de culture contenant une sous-couche de cellules AK-D,
- parasitémie initiale des subcultures : 0,01%,
- hématocrite des subcultures : environ 5%, soit un apport de 100 µl de suspension d'hématies fraîches non parasitées et d'environ 0,9 ml de milieu de culture liquide,
- pH du milieu de culture : 7,3,
- incubation dans une chambre d'incubation à bougie ou dans un incubateur à régulation d'O₂ et CO₂, sous une atmosphère enrichie en CO₂ (3 à 7%) et appauvrie en O₂ (5 à 17%) et à une température de 37°C.

Dans ces conditions, le pourcentage d'hématies parasitées présentes dans le milieu de culture est au moins égal à 1% et peut atteindre des valeurs 10 fois supérieures après 48 heures d'incubation.

L'incubation est arrêtée. Les hématies et le milieu de culture sont récupérés et sont soumis à une dilution propre à permettre l'ensemencement de nouveaux puits de culture avec une suspension d'hématies parasitées à 0,01% et l'incubation de ces hématies.

### 5) Conservation des hématies parasitées recueillies au ternie d'une incubation

Les hématies parasitées recueillies au terme d'une incubation peuvent être, soit utilisées pour entretenir la culture desdits parasites par des subcultures dans les conditions précisées ci-dessus, soit être congelées comme décrit au point 1) de l'Exemple 2 et conservées dans l'azote liquide.

### 6) Croissance des parasites Babesia canis au cours d'une culture en continue

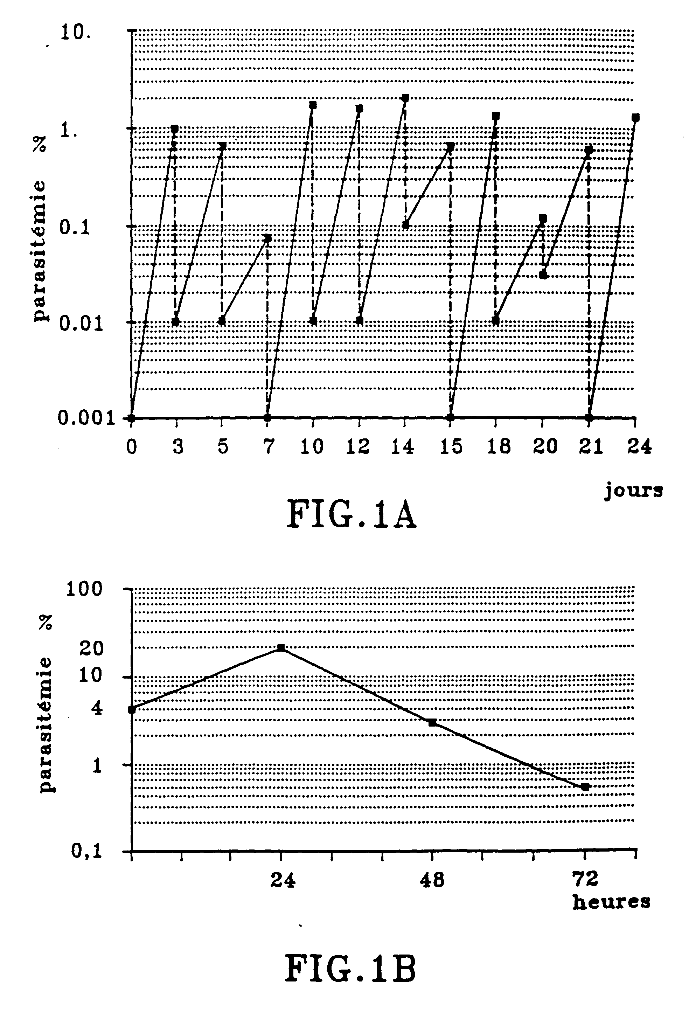

La Figure 1A montre la croissance parasitaire obtenue au cours d'une culture en continue de parasites *Babesia canis* conforme à l'Invention.

L'évolution de la parasitémie représentée sur la Figure 1A a été établie en initialisant une culture de *Babesia canis* avec un pourcentage d'hématies parasitées égal à 0,001% et en procédant à des subcultures avec une parasitémie soit de 0,001% (J7, J15, J21), soit de 0,01% (J3, J5, J10, J12, J18), soit de 0,03% (J20), soit encore de 0,1% (J14). Les pointillés correspondent aux dilutions des hématies parasitées dans des hématies non parasitées propres à diminuer la parasitémie. Les conditions de culture étaient par ailleurs conformes à celles décrites ci-avant.

Cette Figure 1A montre que non seulement le procédé de culture conforme à l'Invention permet d'obtenir à partir de pourcentages d'hématies parasitées très faibles (<0,1%) une croissance parasitaire élevée, mais aussi qu'il est propre à permettre un maintien de cette croissance au cours d'une culture en continue.

A titre de comparaison, la Figure 1B montre la croissance de parasites *Babesia canis* obtenue lors d'une culture *in vitro* réalisée selon le protocole décrit par MOREAU et SOULA (Bull. Soc. Sci. Vét. et Méd. comparée, 1979, 81, n°5). Une culture de *Babesia canis* initiée avec un pourcentage d'hématies parasitées de 4% permet d'obtenir un accroissement du taux d'hématies parasitées pendant les 24 premières heures (jusqu'à 20%) mais celui-ci chute dès la 24ème heure pour atteindre une valeur inférieure à la parasitémie initiale aux environs de la 48ème heure.

### EXEMPLE 3 : ATTENUATION DE LA VIRULENCE D'UNE SOUCHE DE BABESIA CANIS

Une souche virulente de *Babesia canis* dont l'inoculation par voie intra-veineuse à des chiens entraîne l'apparition de signes d'infestation massive et la mort en huit jours a été cultivée pendant 40 jours dans les conditions de culture décrites dans l'Exemple 2.

A l'issue de la culture, l'atténuation de la virulence de cette souche a été testée par inoculation, également par voie intra-veineuse, à des chiens sensibles.

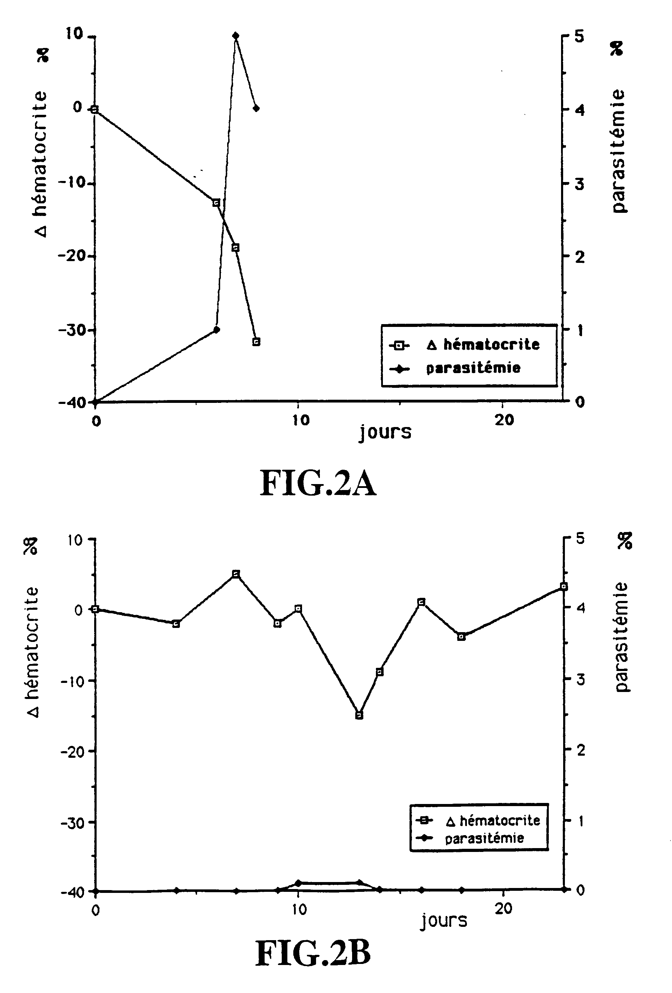

Les Figures 2A et 2B montrent l'évolution de la valeur de l'hématocrite et de la parasitémie intra-veineuse présentés par les chiens ayant respectivement reçu la souche avant qu'elle ne soit cultivée et cette même souche après une culture de 40 jours.

Comme le montre la Figure 2A, l'inoculation de la souche avant culture (Jour 0 sur l'axe des abscisses) se traduit par une chute de l'hématocrite et l'apparition d'une parasitémie atteignant en quelques jours un taux de 10%. Ces signes biologiques s'accompagnent de signes cliniques tels qu'une anorexie, une adynamie, une hyperthermie, une hématurie et des troubles neurologiques aboutissant à la mort des chiens au Sème jour en l'absence d'un traitement curatif.

Lorsque la même souche est inoculée à des chiens sensibles après avoir été préalablement cultivée pendant 40 jours selon le procédé de culture conforme à l'Invention, les chiens présentent, comme visible sur la Figure 28, une baisse discrète de l'hématocrite qui s'accompagne de l'apparition d'une parasitémie intra-veineuse très faible. Cette dernière disparaît spontanément en quelques jours tandis que la valeur de l'hématocrite se corrige. Les chiens ne présentent, par ailleurs, aucun signe clinique d'une infection à *Babesia.*

### EXEMPLE 4 : OBTENTION ET SELECTION D'UNE LIGNEE CLONALE DE BABESIA CANIS

### 1) Obtention d'une lignée clonale

Le clonage de parasites *Babesia canis* peut être réalisé à partir d'hématies parasitées provenant d'une culture continue comme décrite au point 4) de l'Exemple 2.

Pour ce faire, les hématies et le milieu de culture liquide recueillis au terme d'une culture sont soumis à une centrifugation à 1000g pendant 10 minutes à température ambiante. Le surnageant est éliminé et la parasitémie des culots est déterminée.

Le culot est alors soumis à une nouvelle centrifugation à 1500g pendant 3 minutes à température ambiante. Le surnageant est éliminé et le culot est remis en suspension.

A l'aide d'une cellule de numération, on détermine le nombre d'hématies présentes dans un µl et on calcule le nombre d'hématies parasitées par µl de culot.

Ce dernier est dilué dans un volume de milieu de culture liquide de sorte à obtenir une hématie parasitée dans 300 µl de suspension.

On dépose dans chaque puits d'une plaque de culture de 96 puits contenant un tapis de cellules AK-D, 100 µl de ladite suspension de sorte à déposer une hématie parasitée tous les 3 puits et on ajuste l'hématocrite de la culture à 2,5% en ajoutant aux puits un volume approprié de suspension d'hématies fraîches non parasitées.

La plaque de 96 puits est mise à incuber comme précédemment décrit dans une chambre d'incubation à bougie sous une atmosphère enrichie en CO₂ et appauvrie en O₂ et à une température de 37°C.

A J2, on ajoute à chaque puits 100 µl de milieu de culture liquide. On ajuste l'hématocrite à 5% par l'ajout d'un volume approprié de suspension d'hématies fraîches non parasitées et la plaque de culture est remise en chambre d'incubation.

Tous les deux jours, de J4 à J12, on vérifie par un frottis l'apparition de parasites dans chaque puits :
- les cultures dont la parasitémie est négative sont soumises à une dilution au 1/2, transférées sur de nouvelles sous-couches de cellules AK-D et replacées en chambre d'incubation.
- les cultures dont la parasitémie est positive peuvent alors être soumises à une culture en continue propre à obtenir une amplification du clone ainsi obtenu, dans les conditions décrites au point 4) de l'Exemple 2.

Répétée deux fois, cette procédure a permis d'obtenir des clones dit "tertiaires" de *Babesia canis,* c'est à dire des lignées homogènes de parasites, issues chacunes d'un seul parasite et dont les propriétés sont stables *in vitro* et *in vivo.*

Les clones peuvent être congelés comme décrit au point 1) de l'Exemple 1 et conservés dans l'azote liquide.

### 2) Sélection d'une lignée clonale

La sélection d'une lignée clonale, en vue de son utilisation pour la fabrication de vaccins, utilise trois critères :
- son avirulence *in vivo* (absence de symptômes spécifiques d'une babésiose dans un délai de 10 jours après inoculation de la lignée clonale à un animal sensible),
- son rythme de croissance en culture *in vitro,*
- son profil génétique établi par la méthode des empreintes génétiques.

C'est sur la base de ces critères que la Demanderesse a sélectionné la lignée clonale de *Babesia canis* PIII. Celle-ci a été déposée auprès de l'EUROPEAN COLLECTION OF ANIMAL CELL CULTURES en date du 6 Septembre 1994 et identifiée dans cette Collection sous le numéro 94090611.

### EXEMPLE 5 : PREPARATION D'UN VACCIN CONTRE LA BABESIOSE CANINE

Un vaccin vivant atténué contre la babésiose canine peut être préparé avec des hématies parasitées issues :
- soit de la culture d'une souche de parasites *Babesia canis* naturellement avirulents ou peu virulents;
- soit de la culture d'une souche de *Babesia canis* naturellement ayant une virulence plus ou moins prononcée mais dont la virulence aura été préalablement atténuée ;
- soit, enfin, de la culture d'une lignée clonale de *Babesia canis* telle que, par exemple, la lignée clonale PIII, ayant une virulence extrêmement faible.

Les doses vaccinales sont préparées à partir d'hématies parasitées congelées que l'on dilue dans un véhicule acceptable pharmacologiquement tel que, par exemple, du NaCl à 3,5%.

Le vaccin peut être administré aux chiens par voie sous-cutanée, intra-musculaire ou intra-veineuse. La primo-vaccination comporte deux injections à 3 semaines d'intervalle et doit être complétée par un rappel annuel.

### EXEMPLE 6 : IMMUNISATION PAR UN VACCIN CONFORME A l'INVENTION

### 1) Vaccin préparé à partir d'une souche atténuée

L'efficacité d'un vaccin préparé à partir d'une souche atténuée a été mise en évidence par l'administration, à des chiens préalablement vaccinés, de souches virulentes prélevées chez des chiens souffrant de babésiose et originaires de différents départements Français.

Les souches virulentes inoculées et leur origine géographique sont précisées dans le tableau 1 ci-après :

**TABLEAU 1 :**

| **ORIGINE** | **SOUCHES** |
|---|---|
| GIRONDE | Bo*, SG* |
| LANDES | MA*, MB*, MD* |
| LOT-et-GARONNE | VLA |
| HAUTE GARONNE | VB |
| PYRENEES ATLANTIQUES | NA*, NR, NS, NT |
| HAUTES-PYRENEES | GA |
| BAS-RHIN | G2, G4, G6, G8, G10, G12 |

Les souches accompagnées d'un astérisque sont extrêmement virulentes entraînant la mort des chiens infestés.

Le tableau 2 ci-après présente les résultats de cette étude :

**TABLEAU 2 :**

| **SOUCHES INOCULEES** | **EFFICACITE VACCINALE** |
|---|---|
| Bo* | + |
| G2 + G4 + G6 | + |
| G8 + G10 + G12 | + |
| VLA + VB + GA | + |
| NR + NS + NT | + |
| SG* + MB* + NA* | + |
| MD* + MA* | + |

### 2) Vaccin préparé à partir de la lignée clonale PIII

L'efficacité de ce vaccin a été mise en évidence par l'administration par voie intra-veineuse d'une souche particulièrement virulente de *Babesia canis* (souche d'origine bordelaise) chez des chiens ayant reçu, par voie intra-veineuse, 21 jours avant cette administration, une dose vaccinale préparée à partir de la lignée clonale PIII.

L'immunisation a été appréciée par la surveillance de trois critères : l'apparition d'une parasitémie intra-veineuse, l'évolution de la valeur de l'hématocrite et l'état général des chiens.

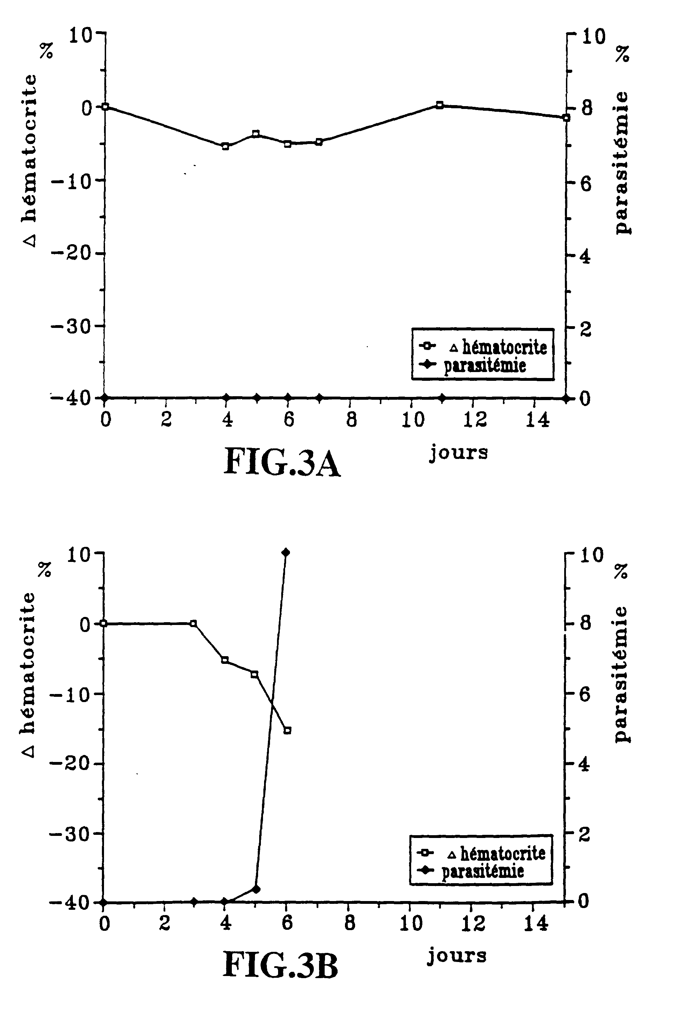

Comme visible sur la Figure 3A, la parasitémie intra-veineuse des chiens reste nulle au cours des quatorze jours suivant l'administration de la souche virulente (Jour 0 sur l'axe des abcisses) et la valeur de l'hématocrite ne subit pas de variations significatives au cours de la même période. En outre, les chiens ne manifestent aucun symptome d'une infection à *Babesia canis.*

A titre de comparaison, la Figure 3B montre l'immunisation obtenue, dans les mêmes conditions et contre la même souche virulente, à l'aide d'un vaccin inactivé de l'Art Antérieur commercialisé sous le nom de PIRODOG® par la Société RHONE MERIEUX. Les chiens ont été vaccinés par voie sous-cutanée conformément au protocole de vaccination recommandé par le fabricant.

Suite à l'administration de la souche virulente (Jour 0 sur l'axe des abcisses), ont été observées une chute de l'hématocrite dès le 3ème jour et une élévation brutale de la parasitémie intra-veineuse des chiens à partir du 4ème jour s'accompagnant sur le plan clinique de signes nerveux et d'une hématurie et aboutissant à la mort des chiens au 6ème jour.

En outre, l'efficacité de ce vaccin atténué a été testée vis-à-vis d'un grand nombre de souches prélevées chez des chiens infectés par *Babesia* et vivant dans différentes régions de France.

Ces souches ainsi que leur origine géographique sont présentées dans le tableau 3 ci-après.

**TABLEAU 3 :**

| **GIRONDE** | **LANDES** | **LOT-ET-GARONNE** | **PYRENEES ATLANTIQUES** | **PARIS** |
|---|---|---|---|---|
| Bo | MA | VLA | FB | P |
| SA | MB | VLB | FC | **BAS-** |
| SAC | MC | VLC | NA | **RHIN** |
| SAD | MD | VLG | NB | G1 |
| SAE | ME | VLH | NC | G2 |
| SAF | MF | VLI | NE | G3 |
| SAG | MI | VLJ | NF | G4 |
| SAH | MJ | VLK | NG | G5 |
| SAL | MK | VLL | NH | G6 |
| SAM | ML | VLM | NN | G8 |
| SAJ | MN | VLN | NO | G10 |
| SAK | MO | | NP | G12 |
| SAL | MP | **HAUTE** | NQ | G13 |
| SB | MQ | **GARONNE** | NR | G14 |
| SC | MR | VA | NS | G15 |
| SE | MS | VB | NT | G16 |
| SG | VE | VD | | G17 |
| SH | | | **HAUTES-** | G18 |
| SJ | | | **PYRENNES** | G19 |
| SK | | | GA | G20 |
| SL | | | | G21 |
| SN | | | **AUDE** | G22 |
| SQ | | | CA | G23 |
| SR | | | | G24 |
| SS | | | | G25 |
| ST | | | | G26 |
| SU | | | | G27 |
| SV | | | | G28 |
| SW | | | | G29 |
| SX | | | | G30 |
| SCA | | | | G31 |
| SCB | | | | G32 |
| SCC | | | | G33 |
| SCD | | | | G35 |
| | | | | G36 |
| | | | | G38 |
| | | | | G39 |
| | | | | G40 |

Ces tests ont permis de montrer que l'administration d'un vaccin préparé à partir de la lignée clonale PIII confère une protection vis-à-vis de 100% des souches françaises testées.

### EXEMPLE 7 : OBTENTION D'UNE SONDE OLIGONUCLEOTIDIQUE DE L'ADN DE LA LIGNEE CLONALE PIII ET CARACTERISATION MOLECULAIRE DE CETTE LIGNEE

Les techniques récentes de biologie moléculaire permettent une identification génétique très fine reposant sur le polymorphisme de l'ADN au niveau de certaines séquences, notamment de séquences répétées plusieurs fois dans le génome (et souvent de nature répétitive).

Dans *Plasmodium falciparum*, autre parasite intra-érythrocytaire et agent du paludisme, plusieurs de ces séquences ont été isolées et ont permis de mettre en évidence un polymorphisme des profils de restriction permettant de différencier des clones parasitaires les uns des autres. (GOMAN et al., Mol. Biochem. Parasitol., 1982, 5, 391-400)

C'est une démarche similaire que la Demanderesse a suivie pour identifier et caractériser le clone PIII de *Babesia canis.*

### 1) Extraction de l'ADN des parasites du clone PIII

Des hématies parasitées recueillies au terme d'une culture ont été soumises, après centrifugation, à une lyse à l'aide de PBS (0,137 mM NaCl, 10 mM Na₂HPO₄, 3,2 mM KH₂PO₄, pH 7,4), additionné de 0,02% de saponine, puis soumises à une centrifugation à 400g.

Les parasites recueillis dans le surnageant ont été lavés avec du PBS et centrifugés à 3000g pour éliminer l'hémoglobine et les membranes. Les parasites ont été lysés dans un tampon TE comprenant 10 mM de Tris-HCl et 1 mM d'acide éthylène-diaminetétraacétique (EDTA) de pH 8, additionné de 0,5% de SDS. Les lysats ont été mis à incuber toute une nuit en présence de protéinase K (1 mg/ml) et soumis à deux extractions successives à l'aide d'un mélange de phénol -chloroforme - alcool isoamylique (25:24:1), puis à une extraction à l'aide de chloroforme. L'ADN présent dans la phase aqueuse a été précipité par l'addition de 3 volumes d'éthanol à température ambiante puis séché, remis en suspension dans du tampon TE, traité par la ribonucléase A (100 µg/ml), puis par la protéinase K (200 µg/ml).

Cette suspension a été soumise à 3 extractions comme précédemment, précipitée, séchée et remise en suspension dans du tampon TE avant d'être soumise à une dialyse en présence de tampon TE sur un disque de cellulose de porosité 0,05 µm.

### 2) Préparation des sondes

L'ADN génomique extrait de parasites du clone PIII a été digéré par la DNaseI en présence de MnCl₂ (1 à 10 mM) pour obtenir des fragments compris entre 0,5 et 2 kbp. Après avoir pourvu les fragments d'ADN d'extrémités franches, des segments de liaison *Not*I ont été ajoutés, l'ADN a été digéré par l'enzyme de restriction *Not*I, puis séparé des segments de liaison digérés et des fragments de séquences inférieures ou égales à 2 kb sur Biogel A50M (PHARMACIA LKD). L'ADN lié a été inséré dans le vecteur Lambda ZAP II (STRATAGENE) et les phages recombinants obtenus ont été amplifiés dans des bactéries *Escherichia coli* XL1 Blue strain.

15000 phages recombinants correspondant à un centième de la banque ont été criblés par hybridation sur membranes de nylon avec de l'ADN total de parasites du clone PIII marqué selon la technique décrite par FEINBERG et VOGELSTEIN (Analyt. Biochem., 1983, 132, 6) à hauteur de 1.10⁹ cpm par µg avec (α³²P) dATP. Les hybridations ont été réalisées toute une nuit à 65°C en présence de 6xSSC, d'une solution 5x de Denhardt, de 100 µg/ml de sperme de saumon dénaturé et 0,5% de SDS. Puis, les membranes ont été lavées deux fois par du 2xSSC additionné de 0,5% de SDS à température ambiante pendant 15 minutes et deux fois par du 0,1xSSC additionné 0,5% de SDS à 65°C pendant 20 minutes et autoradiographiées toute une nuit à -70°C.

36 phages très fortement radiomarqués, susceptibles de contenir des séquences répétées et/ou répétitives ont été sélectionnés. Les inserts génomiques ont été retrouvés par un système d'excision *in vivo* dans le vecteur plasmidique pBluescript® II (SHORT et al., Nucleic Acids Res., 1988, 16, 7583-7600) et récupérés par digestion de l'ADN plasmidique par l'enzyme de restriction *Not*I. Après électrophorèse sur gel d'agarose 1,2%, les inserts ont été purifiés en utilisant des colonnes Spin X (COSTAR).

4 inserts correspondant à 4 familles différentes ont été radiomarqués selon la technique décrite par FEINBERG et VOGELSTEIN et utilisés comme sondes dans des analyses par Southern blot des polymorphismes de longueur des fragments de restriction.

### 3) Analyse des polymorphismes de longueur des fragments de restriction

Différentes souches de parasites d'origines géographiques diverses ont été cultivées par le procédé de culture *in vitro* conforme à l'Invention pour obtenir un nombre de parasites suffisant pour permettre l'extraction de leur ADN.

L'extraction de l'ADN de la lignée clonale PIII comme de ces souches a été réalisée selon la méthode décrite au point 1) du présent Exemple, après une lyse des hématies parasitées.

La concentration de l'ADN a été déterminée par une mesure en spectrophotométrie et son intégrité vérifiée sur gel d'agarose. Les ADN ont été soumis à une digestion totale au moyen d'une ou plusieurs enzymes de restriction (*Ase*I*, Ase*I/*Bam*HI, *Eco*RI ...) et les produits de digestion ont été séparés par électrophorèse en gel d'agarose 0,8% avant d'être transférés sur des membranes de nylon en présence de 20xSSC.

Les membranes ont été soumises à une hybridation par les sondes marquées précitées dans des conditions analogues décrites ci-avant, à l'exception du milieu d'hybridation constitué de 50% de formamide et de 1% de SDS.

Le lavage final a été réalisé à l'aide de 0,1xSSC additionné de 0,5% ou 1% de SDS.

### 4) Résultats

Les expériences d'hybridation avec les 4 sondes testées ont confirmé que celles-ci sont répétées dans le génome des souches, validant ainsi l'hypothèse sur laquelle était fondée leur isolement.

L'une de ces sondes a été retenue en raison de ce qu'elle permet d'obtenir les profils génomiques les plus facilement interprétables, tant au niveau de l'intensité du signal que du nombre et de la répartition des fragments d'ADN obtenus par digestion par des enzymes de restriction telles que la combinaison *Ase*I/*Bam*HI.

Cette sonde présente la séquence suivante :

Cette sonde est spécifique de l'ADN de *Babesia canis* et sensible, offrant un seuil de détection d'environ 10 à 20 pg, ce qui devrait correspondre par analogie avec *Plasmodium falciparum* à 1000 à 2000 parasites.

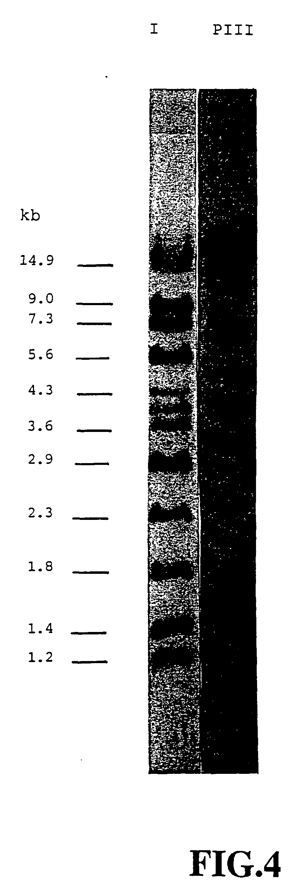

La Figure 4 montre le profil génomique obtenu après une double digestion par les enzymes de restriction *Ase*I/*Bam*HI et une hybridation par la sonde décrite ci-avant, de l'ADN des *Babesia* de la lignée clonale PIII directement issus d'une culture *in vitro* (profil désigné PIII) réalisée dans les conditions décrites dans l'Exemple 2. L'indicateur de poids moléculaire est signalé par la lettre I.

Dans ces conditions, l'ADN génomique de la lignée clonale PIII comprend 16 fragments de restriction respectivement d'environ 17 kb, d'environ 16 kb, d'environ 15 kb, de 13 kb, de 11,5 kb, de 7,1 kb, de 6,6 kb, de 4,8 kb, de 3,8 kb, de 3,6 kb, de 3,15 kb, de 2,3 kb, de 1,5 kb, de 1,4 kb, de 0, 9 kb et de 0,6 kb.

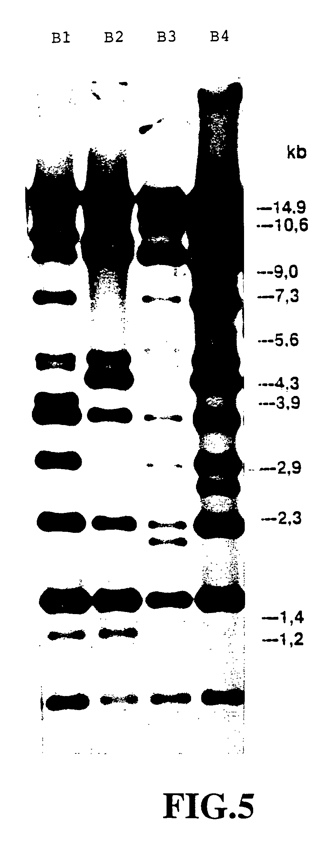

La Figure 5 montre les profils génomiques obtenus après une double digestion par les enzymes de restriction *Ase*I/*Bam*HI de l'ADN des *Babesia* de souches prélevées sur des chiens souffrant d'une babésiose et originaires d'une même région (profils désignés B1, B2, B3 et B4), et hybridation par la sonde décrite précédemment.

Comme le montre cette Figure 5, les souches prélevées chez des chiens affectés d'une babésiose, bien que ces derniers soient originaires de la même région, présentent des profils génomiques différents et spécifiques, confirmant ainsi le pouvoir discriminant de la sonde sélectionnée.

L'utilisation d'autres enzymes de restriction générerait d'autres profils génomiques qui seraient tout aussi spécifiques des souches de *Babesia.*

### EXEMPLE 8 : DETECTION D'UNE INFECTION ASYMPTOMATIQUE A BABESIA CANIS

Il a été montré que les chiens sont souvent porteurs d'une infection asymptomatique à *Babesia canis* non détectable au microscope. Ceci a été établi dans le passé par inoculation du sang de ces chiens à des animaux qui ont développé la maladie.

Le procédé de culture conforme à l'Invention a une sensibilité telle qu'il permet de révéler des infections asymptomatiques à parasitémie très faible.

Une étude a été réalisée sur 43 chiens cliniquement sains vivant dans un biotope très favorable à *Dermacentor reticulatus*, tique vecteur des parasites *Babesia canis*, et donc très exposés au risque de transmission d'une Babésiose canine. Ces chiens avaient, préalablement à cette étude, fait l'objet d'une prophylaxie contre les tiques (balnéation hebdomadaire de dimpylate à 0,5% de Mars à Octobre).

Ces chiens ont été soumis à deux prélèvements sanguins, dans un intervalle de 2 à 5 mois, au cours des six mois suivant l'arrêt du traitement prophylactique.

Ces prélèvements ont fait l'objet, d'une part, d'un examen direct sur la présence d'une parasitémie par microcospie selon des techniques classiquement utilisées en parasitologie et, d'autre part, d'une culture *in vitro* conformément au procédé de culture de la présente Invention.

### 1) Recherche d'une parasitémie par culture in vitro

La recherche d'une parasitémie par culture *in vitro* a été réalisée selon le protocole suivant : les prélèvements sanguins ont été soumis à une centrifugation à 2200g pendant 5 minutes, suivie de deux lavages du culot obtenu par du RPMI 1640 complémenté à la même vitesse de centrifugation. Le surnageant a été éliminé.

Dans les puits d'une plaque de 24 puits, le milieu de culture de cellules AK-D fraîchement repiquées a été remplacé par 1 ml de milieu de culture de *Babesia,* 50 µl du culot sanguin à tester et 50 µl d'une suspension d'hématies fraîches diluées à 50% dans du RPMI 1640 complémenté. Les plaques de culture ont été mises à incuber dans les mêmes conditions que celles décrites dans l'Exemple 2.

L'apparition d'une parasitémie a été suivie par réalisation d'un frottis sur les cultures toutes les 48 heures, accompagné d'une dilution des cultures au 1/2 dans le cas où le frottis se révélait être négatif et ce, jusqu'au 10ème jour.

### 2) Résultats

L'examen microscopique des 86 prélèvements sanguins n'a pas permis de mettre en évidence la présence de parasites *Babesia canis.*

Toutefois, 20 mises en culture sur 86 ont révélé la présence de parasites, c'est à dire que 14 chiens sur 43, soit un tiers de l'effectif, ont été confirmés au moins une fois comme porteurs sains du parasite.

Ainsi que le montre cette étude, l'utilisation du procédé de culture *in vitro* des *Babesia* permet de détecter le portage de ces parasites chez des animaux ne présentant aucun symptôme d'une infection et chez lesquels un examen direct par microscopie ne permet pas de déceler une parasitémie.

### EXEMPLE 9 : CULTURE DE PARASITES BABESIA CANIS EN PRESENCE DE CELLULES C32

Une culture *in vitro* de parasites *Babesia canis* a été réalisée conformément à l'Invention en utilisant des cellules C32 (cellules de mélanome humain) provenant de la souche déposée auprès de l'AMERICAN TYPE CULTURE COLLECTION sous le numéro CRL 1585.

Ces cellules sont capables, tout comme les cellules AK-D, de se multiplier dans un milieu approprié tel que celui décrit au point 2) de l'Exemple 1 et de constituer des sous-couches cellulaires propres à servir de support à la culture de parasites *Babesia.*

Les sous-couches de cellules C32 ont été préparées à partir de cellules congelées selon le protocole décrit au point 3) de l'Exemple 1.

La culture des parasites *Babesia canis* a été initiée à partir d'hématies parasitées comme décrit au point 3) de l'Exemple 2, puis poursuivie en continue en procédant à des subcultures avec une parasitémie soit de 0,001%, soit de 0,005%, soit de 0,01%, soit encore de 0,1%.

Les conditions dans lesquelles ces subcultures ont été réalisées (hématocrite, pH, température, teneurs en O₂ et en CO₂, ...) sont identiques à celles définies au point 4) de l'Exemple 2.

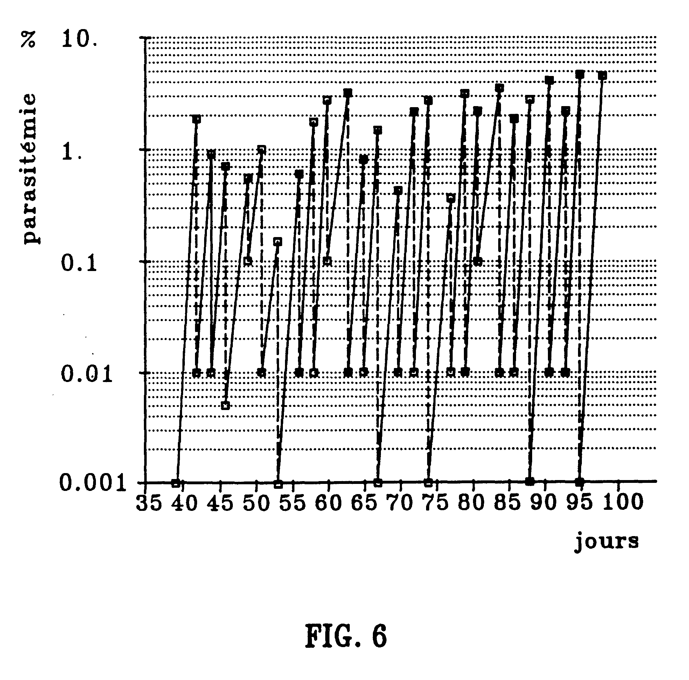

La Figure 6 montre la croissance parasitaire obtenue entre le 38ème jour et le 97ème jour, soit pendant une durée de 59 jours, d'une culture en continue de parasites *Babesia canis* réalisée en présence de cellules C32 dans ces conditions.

## Revendications

1. Procédé de culture *in vitro* de parasites du genre *Babesia* comprenant au moins une incubation d'hématies parasitées par lesdits parasites en présence d'hématies homologues non parasitées dans un milieu de culture convenable et des conditions de culture appropriées, **caractérisé en ce que** la ou lesdites incubations sont réalisées en présence de cellules de support.

2. Procédé de culture *in vitro* de parasites du genre *Babesia* selon la Revendication 1, **caractérisé en ce que** lesdites cellules sont des cellules animales issues de lignées immortelles.

3. Procédé de culture *in vitro* de parasites du genre *Babesia* selon la Revendication 2, **caractérisé en ce que** lesdites cellules sont des cellules AK-D.

4. Procédé de culture *in vitro* de parasites du genre *Babesia* selon l'une quelconque des Revendications précédentes, **caractérisé en ce que** le milieu de culture est un milieu liquide du type RPMI 1640, MEM, M199, WILLIAMS ou autre, additionné de 2 à 40% de sérum homologue ou hétérologue décomplémenté et/ou d'un substitut de sérum et, facultativement, de facteurs de croissance tels que l'hypoxanthine d'antibiotiques tels que la gentamycine, la pénicilline ou la streptomycine, d'antifongiques ou d'antioxydants, ledit milieu de culture étant en outre tamponné de façon à maintenir le pH entre 7 et 7,7.

5. Procédé de culture *in vitro* de parasites du genre *Babesia* selon l'une quelconque des Revendications précédentes, **caractérisé en ce que** le rapport entre le volume des hématies présentes dans la culture et le volume du milieu de culture est compris entre 0,1 et 50% et, de préférence, entre 2,5 et 10%.

6. Procédé de culture *in vitro* de parasites du genre *Babesia* selon l'une quelconque des Revendications précédentes, **caractérisé en ce qu'**il comprend, pour la culture en continue desdits parasites, plusieurs incubations successives.

7. Procédé de culture *in vitro* de parasites du genre *Babesia* selon l'une quelconque des Revendications précédentes, **caractérisé en ce que** la première incubation est réalisée au moyen d'hématies parasitées ou de zoïtes obtenus à partir du sang d'animaux infectés à *Babesia.*

8. Procédé de culture *in vitro* de parasites du genre *Babesia* selon l'une quelconque des Revendications précédentes, **caractérisé en ce qu'**il comprend en outre la conservation à basse température des hématies parasitées dans un milieu comprenant un cryopréservant tel que le diméthylsulfoxyde et une macromolécule à fort pouvoir osmotique telle que la polyvinylpyrrolidone.

9. Procédé de culture *in vitro* de parasites du genre *Babesia* selon l'une quelconque des Revendications précédentes, **caractérisé en ce que** les parasites sont choisis parmi les espèces *Babesia canis, Babesia gibsoni, Babesia bovis, Babesia bigemina, Babesia major, Babesia divergens, Babesia equi. Babesia caballi, Babesia ovis* ou *Babesia microti.*

10. Procédé d'atténuation *in vitro* de la virulence de parasites du genre *Babesia,* **caractérisé en ce qu'**il comprend la culture desdits parasites par le procédé de culture *in vitro* selon l'une quelconque des Revendications 1 à 8 pendant au moins 8 jours.

11. Procédé d'atténuation *in vitro* de la virulence de parasites du genre *Babesia* selon la Revendication 10, **caractérisé en ce que** la culture des parasites est réalisée pendant un temps compris entre 20 et 60 jours.

12. Procédé d'atténuation *in vitro* de la virulence de parasites du genre *Babesia* selon la Revendication 10 ou la Revendication 11, **caractérisé en ce que** les parasites sont choisis parmi les espèces *Babesia canis, Babesia gibsoni, Babesia bovis, Babesia bigemina, Babesia major, Babesia divergens, Babesia equi, Babesia caballi, Babesia ovis* ou *Babesia microti.*

13. Procédé de clonage *in vitro* de parasites du genre *Babesia*, **caractérisé en ce qu'**il comprend :
a) l'isolement d'un seul parasite à partir d'une suspension d'hématies parasitées ou d'une population de zoïtes,
b) la culture du parasite isolé par le procédé de culture *in vitro* selon l'une quelconque des Revendications 1 à 8,
les étapes a) et b) étant effectuées une ou plusieurs fois jusqu'à l'obtention d'une lignée homogène de parasites.

14. Procédé de clonage *in vitro* de parasites du genre *Babesia* selon la Revendication 13, **caractérisé en ce que** l'isolement du parasite est réalisé par une dilution limite de la suspension d'hématies parasitées ou de la population de zoïtes ou au moyen d'un trieur de cellules ou encore par micromanipulation.

15. Procédé de clonage *in vitro* de parasites du genre *Babesia* selon la Revendication 13 ou la Revendication 14, **caractérisé en ce que** les parasites sont choisis parmi les espèces *Babesia canis*, *Babesia gibsoni, Babesia bovis, Babesia bigemina, Babesia major, Babesia divergens, Babesia equi, Babesia caballi, Babesia ovis* ou *Babesia microti.*

16. Lignée clonale de parasites du genre *Babesia,* **caractérisée en ce que** la lignée clonale est la lignée clonale PIII de *Babesia canis,* déposée auprès de l'EUROPEAN COLLECTION OF ANIMAL CELL CULTURES en date du 6 Septembre 1994 et identifiée dans cette Collection sous le n° 94090611.

17. Sonde oligonucléotidique issue de l'ADN de parasites de la lignée clonale PIII de *Babesia canis*, déposée auprès de l'EUROPEAN COLLECTION OF ANIMAL CELL CULTURES en date du 6 Septembre 1994 et identifiée dans cette Collection sous le n° 94090611, **caractérisée en ce qu'**elle comprend tout ou partie de la séquence suivante :

18. Utilisation d'une sonde oligonucléotidique selon la Revendication 17 pour identifier des parasites du genre *Babesia.*

19. Utilisation d'une sonde oligonucléotidique selon la Revendication 18, **caractérisée en ce qu'**elle permet en outre de différencier les souches et/ou lignées clonales de parasites du genre *Babesia.*

20. Vaccin vivant atténué contre une babésiose, **caractérisé en ce qu'**il est propre à induire une protection efficace contre la babésiose canine à *Babesia canis* et **en ce qu'**il est constitué d'hématies parasitées par la lignée clonale PIII de *Babesia canis*, déposée auprès de l'EUROPEAN COLLECTION OF ANIMAL CELL CULTURES en date du 6 Septembre 1994 et identifiée dans cette Collection sous le n° 94090611.

21. Application du procédé de culture *in vitro* de parasites du genre *Babesia* selon l'une quelconque des Revendications 1 à 9 à la production en grand nombre desdits parasites.

22. Application du procédé de culture *in vitro* de parasites du genre *Babesia* selon l'une quelconque des Revendications 1 à 9 à la détection.d'une infection asymptomatique à *Babesia.*

23. Application du procédé d'atténuation *in vitro* de parasites du genre *Babesia* selon l'une quelconque des Revendications 10 à 12 à la préparation de vaccins vivants atténués.

24. Application du procédé de clonage *in vitro* de parasites du genre *Babesia* selon l'une quelconque des Revendications 13 à 15 à la préparation de vaccins vivants atténués.

## Claims

1. Process for *in vitro* culturing of parasites of the genus *Babesia* comprising at least one incubation of erythrocytes parasitised by said parasites in the presence of homologous non-parasitised erythrocytes in a suitable culture medium and under appropriate culture conditions, **characterised in that** the incubation(s) is or are carried out in the presence of support cells.

2. Process for *in vitro* culturing of parasites of the genus *Babesia* according to claim 1, **characterised in that** said cells are animals cells originating from immortal lines.

3. Process for *in vitro* culturing of parasites of the genus *Babesia* according to claim 2, **characterised in that** said cells are AK-D cells.

4. Process for *in vitro* culturing of parasites of the genus *Babesia* according to any one of the preceding claims, **characterised in that** the culture medium is a liquid medium of the type RPMI 1640, MEM, M199, WILLIAMS or the like, to which 2 to 40% of decomplemented homologous or heterologous serum and/or a serum substitute and, optionally, growth factors such as hypoxanthine, antibiotics such as gentamycin, penicillin or streptomycin, antifungal agents or antioxidants have been added, said culture medium further being buffered so as to maintain the pH between 7 and 7.7.

5. Process for *in vitro* culturing of parasites of the genus *Babesia* according to any one of the preceding claims, **characterised in that** the ratio between the volume of erythrocytes present in the culture and the volume of the culture medium is between 0.1 and 50%, preferably between 2.5 and 10%.

6. Process for *in vitro* culturing of parasites of the genus *Babesia* according to any one of the preceding claims, **characterised in that** it comprises a plurality of successive incubations for continuous culture of said parasites.

7. Process for *in vitro* culturing of parasites of the genus *Babesia* according to any one of the preceding claims, **characterised in that** the first incubation is carried out by means of parasitised erythrocytes or zoites obtained from the blood of animals infected with *Babesia.*

8. Process for *in vitro* culturing of parasites of the genus Babesia according to any one of the preceding claims, **characterised in that** it further comprises low temperature preservation of parasitised erythrocytes in a medium containing a cryoprotectant such as dimethylsulphoxide and a macromolecule with a strong osmotic power such as polyvinyl pyrrolidone.

9. Process for *in vitro* culturing of parasites of the genus *Babesia* according to any one of the preceding claims, **characterised in that** the parasites are selected from among the species *Babesia canis, Babesia gibsoni, Babesia bovis, Babesia bigemina, Babesia major, Babesia divergens, Babesia equi, Babesia caballi, Babesia ovis* or *Babesia microti.*

10. Process for attenuating *in vitro* the virulence of parasites of the genus *Babesia,* **characterised in that** it comprises culturing said parasites by the *in vitro* culturing process according to any one of claims 1 to 8, for at least 8 days.

11. Process for attenuating *in vitro* the virulence of parasites of the genus *Babesia* according to claim 10, **characterised in that** the parasites are cultured over a period of between 20 and 60 days.

12. Process for attenuating *in vitro* the virulence of parasites of the genus *Babesia* according to claim 10 or 11, **characterised in that** the parasites are selected from among the species *Babesia canis, Babesia gibsoni, Babesia bovis, Babesia bigemina, Babesia major, Babesia divergens, Babesia equi, Babesia caballi, Babesia ovis* or *Babesia microti.*

13. Process for cloning parasites of the genus *Babesia in vitro,* **characterised in that** it comprises:
a) isolating a single parasite from a suspension of parasitised erythrocytes or a population of zoites,
b) culturing the parasite isolated by the *in vitro* culturing process according to any one of claims 1 to 8, steps a) and b) being carried out one or more times until a homogeneous line of parasites is obtained.

14. Process for *in vitro* cloning of parasites of the genus *Babesia* according to claim 13, **characterised in that** the isolation of the parasite is obtained by a limiting dilution of the suspension of parasitised erythrocytes or the population of zoites or using a cell sorter or by microengineering.

15. Process for *in vitro* cloning of parasites of the genus *Babesia* according to claim 13 or 14, **characterised in that** the parasites are selected from among the species *Babesia canis, Babesia gibsoni, Babesia bovis, Babesia bigemina, Babesia major, Babesia divergens, Babesia equi, Babesia caballi, Babesia ovis* or *Babesia microti.*

16. Clonal line of parasites of the genus *Babesia,* **characterised in that** the clonal line is the clonal line PIII of *Babesia canis*, deposited at the EUROPEAN COLLECTION OF ANIMAL CELL CULTURES on 6th September 1994 and identified in this Collection by no. 94090611.

17. Oligonucleotide probe obtained from the DNA of parasites of the clonal line PIII of *Babesia canis,* deposited at the EUROPEAN COLLECTION OF ANIMAL CELL CULTURES on 6th September 1994 and identified in this Collection by no. 94090611, **characterised in that** it comprises all or part of the following sequence:

18. Use of an oligonucleotide probe according to claim 17 for identifying parasites of the genus *Babesia.*

19. Use of an oligonucleotide probe according to claim 18, **characterised in that** it further enables the strains and/or clonal lines of parasites of the genus *Babesia* to be differentiated.

20. Attenuated live vaccine against babesiosis, **characterised in that** it is capable of inducing effective protection against canine babesiosis with *Babesia canis,* and **in that** it consists of erythrocytes parasitised by the clonal line PIII of *Babesia canis,* deposited at the EUROPEAN COLLECTION OF ANIMAL CELL CULTURES on 6th September 1994 and identified in this Collection by no. 94090611.

21. Use of the process for *in vitro* culturing of parasites of the genus *Babesia* according to any one of claims 1 to 9 in the large-scale production of said parasites.

22. Use of the process for *in vitro* culturing of parasites of the genus *Babesia* according to any one of claims 1 to 9 in the detection of asymptomatic infection with *Babesia.*

23. Use of the process for *in vitro* culturing of parasites of the genus *Babesia* according to any one of claims 10 to 12 in the preparation of attenuated live vaccines.

24. Use of the process for *in vitro* cloning of parasites of the genus *Babesia* according to any one of claims 13 to 15 in the preparation of attenuated live vaccines.

## Patentansprüche

1. Verfahren zur *in vitro*-Kultur von Parasiten der Gattung *Babesia*, umfassend mindestens eine Inkubation von Erythrozyten, die von den Parasiten parasitiert sind, in Gegenwart von nicht-parasitierten homologen Erythrozyten in einem zweckdienlichen Kulturmedium und unter geeigneten Kulturbedingungen, **dadurch gekennzeichnet, dass** die Inkubation oder die Inkubationen in Gegenwart von Trägerzellen durchgeführt wird/werden.

2. Verfahren zur *in vitro*-Kultur von Parasiten der Gattung *Babesia* nach Anspruch 1, **dadurch gekennzeichnet, dass** die Zellen Tierzellen sind, die von unsterblichen Stämmen stammen.

3. Verfahren zur *in vitro*-Kultur von Parasiten der Gattung *Babesia* nach Anspruch 2, **dadurch gekennzeichnet, dass** die Zellen AK-D-Zellen sind.

4. Verfahren zur *in vitro*-Kultur von Parasiten der Gattung *Babesia* nach einem der vorangehenden Ansprüche, **dadurch gekennzeichnet, dass** das Kulturmedium ein flüssiges Medium des Typs RPMI 1640, MEM, M199, WILLIAMS oder ein anderes ist, das mit 2 bis 40 % dekomplementiertem homologen oder heterologen Serum und/oder einem Serumersatzmittel und fakultativ mit Wachstumsfaktoren wie Hypoxanthin, Antibiotika wie Gentamycin, Penicillin oder Streptomycin, mit antifungalen Mitteln oder Antioxidanzien versetzt ist, wobei das Kulturmedium außerdem so gepuffert ist, dass der pH zwischen 7 und 7,7 gehalten wird.

5. Verfahren zur *in vitro*-Kultur von Parasiten der Gattung *Babesia* nach einem der vorangehenden Ansprüche, **dadurch gekennzeichnet, dass** das Verhältnis zwischen dem Volumen der Erythrozyten, die in der Kultur vorliegen, und dem Volumen des Kulturmediums zwischen 0,1 und 50 % und vorzugsweise zwischen 2,5 und 10 % liegt.

6. Verfahren zur *in vitro*-Kultur von Parasiten der Gattung *Babesia* nach einem der vorangehenden Ansprüche, **dadurch gekennzeichnet, dass** es für eine kontinuierliche Kultur der Parasiten mehrere aufeinanderfolgende Inkubationen umfasst.

7. Verfahren zur *in vitro*-Kultur von Parasiten der Gattung *Babesia* nach einem der vorangehenden Ansprüche, **dadurch gekennzeichnet, dass** die erste Inkubation mittels parasitierter Erythrozyten oder Zoiten, die aus Blut von Tieren, die mit *Babesia* infiziert sind, erhalten werden, durchgeführt wird.

8. Verfahren zur *in vitro*-Kultur von Parasiten der Gattung *Babesia* nach einem der vorangehenden Ansprüche, **dadurch gekennzeichnet, dass** es außerdem die Konservierung der parasitierten Erythrozyten bei niedriger Temperatur in einem Medium, welches ein Cryokonservierungsmittel wie Dimethylsulfoxid und ein Makromolekül mit starker osmotischer Kraft wie Polyvinylpyrrolidon umfasst, umfasst.

9. Verfahren zur *in vitro*-Kultur von Parasiten der Gattung *Babesia* nach einem der vorangehenden Ansprüche, **dadurch gekennzeichnet, dass** die Parasiten unter den Arten *Babesia canis, Babesia gibsoni, Babesia bovis, Babesia bigemina, Babesia major, Babesia divergens, Babesia equi, Babesia caballi, Babesia ovis* oder *Babesia microti* ausgewählt werden.

10. Verfahren zur *in vitro*-Abschwächung der Virulenz von Parasiten der Gattung *Babesia,* **dadurch gekennzeichnet, dass** es die Kultur der Parasiten gemäß dem Verfahren zur *in vitro*-Kultur nach einem der Ansprüche 1 bis 8 für mindestens acht Tage umfasst.

11. Verfahren zur *in vitro*-Abschwächung der Virulenz von Parasiten der Gattung *Babesia* nach Anspruch 10, **dadurch gekennzeichnet, dass** die Kultur der Parasiten über eine Zeit durchgeführt wird, die zwischen 20 und 60 Tagen liegt.

12. Verfahren zur in vitro-Abschwächung der Virulenz von Parasiten der Gattung *Babesia* nach Anspruch 10 oder Anspruch 11, **dadurch gekennzeichnet, dass** die Parasiten unter den Arten *Babesia canis, Babesia gibsoni*, *Babesia bovis, Babesia bigemina, Babesia major, Babesia divergens, Babesia equi, Babesia caballi, Babesia ovis* oder *Babesia microti* ausgewählt werden.

13. Verfahren der *in vitro*-Klonierung von Parasiten der Gattung *Babesia,* **dadurch gekennzeichnet, dass** es umfasst:
a) die Isolierung eines einzelnen Parasiten ausgehend von einer Suspension von parasitierten Erythrozyten oder einer Zoiten-Population,
b) die Kultur des isolierten Parasiten nach dem Verfahren zur *in vitro*-Kultur nach einem der Ansprüche 1 bis 8, wobei die Stufen a) und b) ein- oder mehrmals bis zum Erhalt eines homogenen Parasitenstamms durchgeführt werden.

14. Verfahren zur *in vitro*-Klonierung von Parasiten der Gattung *Babesia* nach Anspruch 13, **dadurch gekennzeichnet, dass** die Isolierung des Parasiten durch eine Grenzverdünnung der Suspension der parasitierten Erythrozyten oder der Zoitenpopulation oder mit Hilfe eines Zellsortierers oder auch durch Mikromanipulation durchgeführt wird.

15. Verfahren zur *in vitro*-Klonierung von Parasiten der Gattung *Babesia* nach Anspruch 13 oder Anspruch 14, **dadurch gekennzeichnet, dass** die Parasiten unter den Arten *Babesia canis, Babesia gibsoni, Babesia bovis, Babesia bigemina, Babesia major, Babesia divergens, Babesia equi, Babesia caballi, Babesia ovis* oder *Babesia microti* ausgewählt werden.

16. Klonaler Parsitenstamm der Gattung Babesia, **dadurch gekennzeichnet, dass** der klonale Stamm der klonale Stamm PIII von *Babesia canis* ist, der bei der EUROPEAN COLLECTION OF ANIMAL CELL CULTURES am 6. September 1994 hinterlegt wurde und bei dieser Sammlung unter der Nr. 94090611 eingeordnet ist.

17. Oligonucleotidsonde aus der DNA von Parasiten des klonalen Stamms PIII von *Babesia Canis,* der bei der EUROPEAN COLLECTION OF ANIMAL CELL CULTURES am 6. September 1994 hinterlegt wurde und bei dieser Sammlung unter der Nr. 94090611 eingeordnet ist, **dadurch gekennzeichnet, dass** sie die folgende Sequenz ganz oder teilweise umfasst:

18. Verwendung einer Oligonucleotidsonde nach Anspruch 17 zur Identifizierung von Parasiten der Gattung *Babesia.*

19. Verwendung einer Oligonucleotidsonde nach Anspruch 18, **dadurch gekennzeichnet, dass** sie es außerdem ermöglicht, die Stämme und/oder klonalen Stämmen von Parasiten der Gattung *Babesia* zu differenzieren.

20. Abgeschwächter Lebendimpfstoff gegen Babesiose, **dadurch gekennzeichnet, dass** er geeignet ist, einen wirksamen Schutz gegen Hunde-Babesiose, verursacht durch *Babesia canis,* zu induzieren und dass er aus Erythrozyten besteht, die durch den klonalen Stamm PIII von *Babesia canis,* hinterlegt bei der EUROPEAN COLLECTION OF ANIMAL CELL CULTURES am 6. September 1994 und bei dieser Sammlung unter der Nr. 94090611 eingetragen, parasitiert sind.

21. Verwendung des Verfahrens zur *in vitro-*Kultur von Parasiten der Gattung *Babesia* nach einem der Ansprüche 1 bis 9 zur Herstellung einer großen Zahl dieser Parasiten.

22. Verwendung des Verfahrens zur *in vitro*-Kultur von Parasiten der Gattung *Babesia* nach einem der Ansprüche 1 bis 9 zum Nachweis einer asymptomatischen Infektion durch *Babesia.*

23. Verwendung des Verfahrens zur *in vitro*-Abschwächung von Parasiten der Gattung *Babesia* nach einem der Ansprüche 10 bis 12 zur Herstellung von abgeschwächten Lebendimpfstoffen.

24. Anwendung des Verfahrens zur *in vitro*-Klonierung von Parasiten der Gattung *Babesia* nach einem der Ansprüche 13 bis 15 zur Herstellung von abgeschwächten Lebendimpfstoffen.
